# EUROPEAN PATENT APPLICATION

(11) **EP 1 312 376 A1**
(43) Date of publication of application: **21.05.2003**
(21) Application number: 01956846.8
(22) Date of filing: 10.08.2001
(51) Int. Cl.: A61K 38/00

(54) **VASCULAR RELAXATION AGENTS**

(30) Priority: 11.08.2000 JP 2000243873
(71) Applicant: CHUGAI SEIYAKU KABUSHIKI KAISHA, Tokyo, 115-8543 (JP)
(72) Inventor: OHHASHI, Toshio, Matsumoto-shi, Nagano 390-0812 (JP)
(74) Representative: Maschio, Antonio
(86) International application number: JP0106943
(87) International publication number: WO02013852

(57) **Abstract**

To provide vascular relaxation agents and vascular relaxation inhibitors. Vascular relaxation agents comprising PTHrP or a fragment thereof and vascular relaxation inhibitors comprising a substance capable of inhibiting the binding between PTHrP and a receptor thereof.

## Description

### TECHNICAL FIELD

This invention relates to a vascular relaxation agent comprising PTHrP or a fragment thereof and a vascular relaxation inhibitor comprising a substance which can inhibit the binding between PTHrP and a receptor thereof.

### BACKGROUND ART

Parathyroid hormone related protein (hereinafter referred to as "PTHrP"), which is a protein produced by a tumor, is a major causative agent of hypercalcemia. This protein causes tumor-producing hypercalcemia (Humoral hypercalcemia of malignancy, hereinafter referred to as "HHM") by promoting calcium reabsorption in the bone and renal tubule.

It is understood that PTHrP metabolizes bone and minerals as strongly as parathyroid hormone, (hereinafter referred to as "PTH"), since PTHrP was found as causative agent of hypercalcemia. It has been further found that PTHrP is widely present in various tissues which are not involved in mineral metabolism, such as the endocrine gland, blood vessels, smooth muscles of the bladder, smooth muscles of the uterus, mammary gland, epidermis, central nerves. Thereafter, a PTHrP receptor gene was identified, and it was found that PTHrP receptors were also widely expressed in various tissues where PTHrP is expressed (Mok LLS, et al., Endocrinol. Rev. 10:420-436, 1989; Philbrick, W. M. et al., Physiol. Rev. 76: 127-173, 1996; Wysolmerski, J. J. and Stewart, A. F., Annu. Rev. Physiol. 60: 431-460, 1998).

However, it has been unclear what effect PTHrP has upon vessels or lymph ducts.

### DISCLOSURE OF THE INVENTION

It is an object of the present invention to provide relaxation agents, which comprise PTHrP or a fragment thereof, for the vascular, lymph ducts, and medullary arterioles, and relaxation inhibitors, which comprise a substance capable of inhibiting the binding between PTHrP and a receptor thereof, for the vascular, medullary arterioles, and medullary arterioles.

The present inventor has made intensive studies to solve the above problem. As a result, the inventor found that PTHrP or a fragment thereof has relaxation or dilation effect on vessels (lymph ducts and medullary arterioles), and further that a substance capable of inhibiting the binding between PTHrP and a receptor thereof has a vascular relaxation suppressing property, thereby leading to the completion of the present invention.

Namely, according to the present invention, there is provided a vascular relaxation agent comprising PTHrP or a fragment thereof, and in particular, a vascular relaxation agent which relaxes lymph ducts or medullary arterioles.

Further, according to the present invention, there is provided a therapeutic agent, which comprises PTHrP or a fragment thereof, against a disease attributable to vascular contraction. As a disease attributable to vascular contraction, at least one disease selected from the group consisting of myocardial infraction, hypertension, thrombosis, congestive heart failure, and angina pectoris may be given.

Furthermore, according to the present invention, there is provided a vascular relaxation inhibitor comprising a substance capable of inhibiting the binding between PTHrP and a receptor thereof, and in particular a vascular relaxation inhibitor which inhibits the relaxation of lymph ducts or medullary arterioles.

Moreover, according to the present invention, there is provided a therapeutic agent, which comprises a substance capable of inhibiting the binding between PTHrP and a receptor thereof, against diseases attributable to vascular relaxation, lymph duct relaxation, and medullary arteriole relaxation. As a disease attributable to vascular relaxation, at least one disease selected from the group consisting of hypotension, peripheral circulatory failure, and multiple organ failure may be given. As a disease attributable to lymph duct relaxation, at least one disease selected from the group consisting of lymph duct cancer metastasis, lymphedema, lymphadenitis, and intestinal lymphangiectasis may be given. As a disease attributable to medullary arteriole relaxation, at least one disease selected from the group consisting of bone metastasis, osteonecrosis, osteomyelitis, osteoporosis, Paget's disease of the bone, rheumatoid arthritis, and osteomalasia may be given.

Further, according to the present invention, there is provided a method for preparing a perfused medullary arteriole specimen, wherein the method comprises:
(a) collecting a thigh bone from a laboratory animal;
(b) stripping off a surface of the thigh bone so as to expose its bone marrow;
(c) extirpating the medullary arteriole from inside the bone marrow;
(d) mounting the extirpated arteriole on cannulas; and
(e) supplying an internal pressure to the arteriole.
   According to the present invention, there is also provided a method for screening a medullary arteriole relaxation agent and/or relaxation inhibitor, wherein the method comprises: adding a test substance to the specimen obtained by the method for preparing the perfused specimen; and screening the test substance using the specimen's contraction as an index.

In addition, according to the present invention, there is provided a measurement apparatus comprising: a pair of cannulas which are attached on both ends of the vessel to be examined so as to support the vessel; internal pressure application means which supplies an internal pressure to the vessel to be examined via at least one of the cannulas; a vessel chamber filled with a perfusion liquid which contains the vessel supported by the pair of cannulas; and imaging means which takes an image of the vessel contained in the vessel chamber, wherein the measurement apparatus measures the contractions and relaxations of the vessel to be examined by detecting changes in the shape of the vessel which is supported by the pair of cannulas.

Furthermore, according to the present invention, there is provided a method which comprises the steps of: attaching a pair of cannulas on both ends of a vessel to be examined; supporting the vessel by the pair of cannulas; supplying an internal pressure to the vessel to be examined via at least one of cannulas; and measuring the contraction and/or relaxation of the vessel to be examined, which is supported by the pair of cannulas and is immersed in a perfusion liquid filled into a vessel chamber.

Hereinafter, the present invention will be described in detail.

The present invention provides a relaxation agent for vessels and the like based on the finding that parathyroid hormone related protein (hereinafter referred to as "PTHrP") or a fragment thereof relaxes vessels, in particular lymph ducts or medullary arterioles. Further, the present invention provides an agent for inhibiting relaxation of lymph ducts or vessels using a substance capable of inhibiting the binding between PTHrP and a receptor thereof.

### 1. Preparation of PTHrP or a fragment thereof

The whole amino acid sequence (SEQ ID NO:1 , 2 or 3) of PTHrP is known, and its fragment, for example, PTHrP (1-34) can be obtained by peptide synthesis. Alternatively, it is also commercially available.

As a fragment of PTHrP, any fragment such as PTHrP(1-34) and PTHrP(1-36), can be used as long as it contains PTHrP(1-34). Further, variants thereof (comprising several amino acid substitutions, deletions, or additions), amidated products thereof, or the like can also be used. Among these, it is particularly preferable to use PTHrP(1-34).

When peptide synthesis is adopted, PTHrP can be obtained by peptide chemical synthesis which is ordinarily conducted. For conducting peptide chemical synthesis, for example, the Azide method, the Acid chloride method, the Acid anhydride method, the Mixed acid anhydride method, the DCC method, the Activated ester method, the Carbon-imidazole method, or the Oxidization-reduction method may be employed. Further, either solid synthesis method or liquid synthesis method can be employed for the synthesis.

Namely, amino acids which can constitute a peptide of the present invention are condensed and in the case where the resultant product has a protecting group, elimination of this protecting group enables the synthesis of the peptide of interest. Any known condensation method or protecting group elimination method may be employed (Bodanszky, M and M.A. Ondetti, Peptide Synthesis, Interscience Publishers, New York (1966); Schroeder and Luebke, The Peptide, Academic Press, New York (1965); Nobuo IZUMIYA et al., "PEPUCHIDO-GOUSEI NO KISO TO JIKKEN (Basics and Experiments of Peptide Synthesis)," Maruzen (1975), etc.).

After the reaction, peptides of interest are purified by the combination of common purification methods, for example, solvent extraction, distillation, column chromatography, liquid chromatography, and recrystallization. Analysis of whether or not the synthesized peptides are of interest can be carried out by mass spectrometry, nuclear magnetic resonance, electrophoresis, high performance liquid chromatography, and others.

### 2. Substance capable of inhibiting the binding between PTHrP and a receptor thereof

The expression "substance capable of inhibiting the binding between PTHrP and a receptor thereof" (hereinafter referred to as "PTHrP inhibitor") means any one or both of a substance (e.g. anti PTHrP antibodies) which can inhibit PTHrP from being bound to a PTHrP receptor by binding itself to PTHrP and a substance which binds itself to a PTHrP receptor thereby inhibiting PTHrP from binding to a PTHrP receptor. It is noted that based on selective gene splicing, three types of PTHrP are known, which comprise 139, 141, and 173 amino acids, respectively. All the three types have an identical amino acid sequence of up to amino acid at position 139. A sequence comprising 139 amino acids is represented by SEQ ID NO:1, a sequence comprising 141 amino acids is represented by SEQ ID NO:2, and a sequence comprising 173 amino acids is represented by SEQ ID NO:3.

Further, the above PTHrP includes a partial segment at the N-terminal side. Therefore, examples of PTHrP inhibitors include: a substance (e.g., anti PTHrP(1-34) antibodies) which inhibits a peptide (referred to as "PTHrP(1-34)") comprising a sequence of 1 to 34 amino acids relative to the amino acid sequences of the PTHrP from being bound to a PTHrP(1-34) receptor by binding itself to PTHrP(1-34); and a substance (hereinafter referred to as "PTHrP(1-34) inhibitor") which inhibits PTHrP(1-34) from being bound to a PTHrP(1-34) receptor by binding itself to a PTHrP(1-34) receptor.

Examples of the PTHrP inhibitors include: (1) PTHrP receptor antagonists (e.g., PTHrP(1-34) antagonists); (2) PTHrP(1-34) peptides comprising at least one amino acid (e.g. one or several amino acids) substitution, deletion, or addition; and (3) a partial sequence of PTHrP(1-34) peptide (e.g., PTHrP(7-34)). Here, PTHrP(7-34) means a peptide having a sequence comprising from 7 to 34 amino acids relative to the amino acid sequence of PTHrP.

### (1) PTHrP(1-34) antagonist

As a PTHrP(1-34) antagonist, polypeptides or low molecules are used, but it is not limited thereto. Examples of polypeptides which are antagonistically bound to PTHrP(1-34) receptors against PTHrP(1-34) include PTHrP(7-34), PTHrP(8-34), PTHrP(9-34), PTHrP(10-34) or variants thereof (comprising several amino acid substitutions, deletions, and additions), or amidated products thereof. In addition, other examples of peptides having PTHrP(1-34) antagonistic activity are described in: JP Patent Publication (Unexamined Application) No. 7-165790; JP Patent Publication (PCT Translation) No. 5-509098; Peptides(UNITED STATES)1995, 16(6)1031-1037; and Biochemistry(UNITED STATES)Apr.281992, 31(16)4026-4033. Among the described polypeptides, polypeptides (e.g., PTHrP(7-34)) which comprise at least one (e.g. one or several) amino acid deletion, substitution, addition, or insertion and have the same level of PTHrP(1-34) antagonistic activity as the above exemplified polypeptides are also included in PTHrP(1-34) antagonists of the present invention. Further, nitric oxide (NO) synthesis inhibitors are also included in PTHrP(1-34) antagonists. Examples of NO synthesis inhibitors include L-NAME(N^{G}-nitro L-arginine methyl ester), L-Arg(L-arginine), L-NA(N^{G}-nitro L-arginine), and L-NAMA(N^{G}-monomethyl L-arginine).

### (2) Anti-PTHrP antibody

As an anti-PTHrP antibody, for example, a humanized antibody (International Patent Publication No. WO98/13388), a human antibody (International Patent Publication No. WO96/33735), and a chimeric antibody (JP Patent Publication (Unexamined Application) No. 4-228089) may be used. In addition to these, an antibody (#23-57-137-1 antibody) which can be produced with hybridoma #23-57-137-1 may also be used. However, although the antibody may be polyclonal, monoclonal antibody is preferable.

The anti-PTHrP antibody (such as PTHrP(1-34) antibody) to be used in the present invention can be produced by any known method as a polyclonal or monoclonal antibody. As the antibody to be used in the present invention, mammal-derived monoclonal antibody is particularly preferable. The mammal-derived monoclonal antibody includes, in addition to those produced by a hybridoma, those produced by a host, such as a CHO cell, a COS cell, or a BHK cell, and transformed by an expression vector carrying a gene for the antibody by means of genetic engineering.

### 3. Use of PTHrP or a fragment thereof

### (1) Vascular relaxation agent

PTHrP or a fragment thereof (except PTHrP(7-34)) can suppress (i.e. relax) the contraction of blood vessels, lymph ducts, or the like, and therefore, they can be used as vascular relaxation agents, in particular, lymph duct relaxation agents or medullary arteriole relaxation agents. Examples of PTHrP include those represented by SEQ ID NO:1 to 3, and examples of fragments thereof include any fragment, such as PTHrP(1-34) and PTHrP(1-36), comprising PTHrP(1-34).

As used herein, the term "vessel" means a tube for conveying liquid in vivo, and examples thereof include blood vessels for conveying blood (arteries and veins), lymph ducts, and medullary arterioles. The vascular relaxation agent of the present invention has been accomplished by finding, for the first time, that the agent of the present invention causes micro arteries such as lymph ducts or medullary arterioles to be relaxed.

The evaluation method of relaxation or contraction response is conducted based on a value (relative spontaneous contraction suppression) obtained by dividing the number of spontaneous contractions per minute observed after drug administration until returning to even spontaneous contractions by the average number of spontaneous contractions per minute before drug administration. For example, supposing that it takes 3 minutes to return to even spontaneous contractions after drug administration and that the contractions occur 9 times - during that period, the value after drug administration is 3 times/minute. Therefore, if the frequency before drug administration is 15 times/minute, the suppression effect is represented as 20%, which is calculated by dividing 3 by 15 and standardizing the quotient as a percent.

### (2) Therapeutic agent for diseases

According to the present invention, PTHrP or a fragment thereof can be used as a therapeutic agent for diseases attributable to vascular or lymph duct contractions. Examples of the disease attributable to vascular contractions include myocardial infraction, hypertension, thrombosis, congestive heart failure, and angina pectoris. In addition, the onset of either alone or combination of these diseases, or a complication thereof with other diseases may be included.

A therapeutic agent comprising PTHrP or a fragment thereof as an active ingredient for the treatment of diseases attributable to vascular contractions may be administered orally or parenterally, but parenteral administration is preferable. Examples of dosage forms of the therapeutic agent include a transpulmonary agent (e.g., an agent administered with the help of a device such as a nebulizer), a nasogastric agent, a transdermic agent (e.g., ointment, cream), and an injection. Examples of the injection include an intravenous injection (e.g., drops), an intramuscular injection, an intraperitoneal injection and a subcutaneous injection for systemic or topical administration. Further, depending on the age or the conditions of the patient, the route of administration may be selected accordingly. An effective single dose may be selected within the range from 0.001 to 1000 mg per kilogram of the body weight. Alternatively, the dose per patient may be selected in the range from 0.01 to 100000 mg/body, preferably 0.1 to 10000 mg/body, more preferably 0.5 to 1000 mg/body, and much more preferably 1 to 100 mg/body. However, the therapeutic agent is not limited to these dosage ranges.

The therapeutic agent of the present invention may be formulated by any conventional method (Remington's Pharmaceutical Science, latest edition, Mark Publishing Company, Easton, USA). The preparation may further comprise pharmaceutically acceptable carriers and additives.

Examples of such carriers and pharmaceutical additives include water, pharmaceutically acceptable organic solvents, collage, polyvinyl alcohol, polyvinylpyrrolidone, carboxyvinyl polymer, sodium carboxymethyl cellulose, sodium polyacrylate, sodium alginate, water-soluble dextran, carboxymethyl starch sodium, pectin, methylcellulose, ethylcellulose, xantahn gum, gum Arabic, casein, agar, polyethylene glycol, diglycerine, glycerine, propylene glycol, Vaseline, paraffin, stearyl alcohol, stearic acid, human serum albumin (HAS), mannitol, sorbitol, lactose, and surfactants acceptable as pharmaceutical additives.

In practical use, the additive is properly selected from the above members either alone or in combination, depending on the dosage form employed for the inhibitors of the present invention, but not limited thereto. For example, for use as an injectable form, the purified antibody is first dissolved in a solvent (e.g., physiological saline, a buffer, a glucose solution), then an adsorption-preventing agent (e.g., Tween 80, Tween 20, a gelatin, human serum albumin) is added to the obtained solution, and the resultant product can be used. The therapeutic agent of the present invention may also be in a reconstitutable freeze-dried dosage form, which is dissolved before use. For the formulation of the freeze-dried dosage form, excipients such as sugar alcohols (e.g., mannitol, glucose) or sugars may be used.

### 4. Use of a substance capable of inhibiting the binding between PTHrP and a receptor thereof

### (1) Relaxation inhibitor

A substance capable of inhibiting the binding between PTHrP and a receptor thereof can suppress the relaxation of blood vessels, lymph ducts, or the like. That is, when a blood vessel or the like is relaxed due to the presence of a tumor etc., the substance causes the vessel to recover its inherent contraction function. Therefore, it can be used as a vascular relaxation inhibitor, in particular, a lymph duct relaxation inhibitor or a medullary arteriole relaxation inhibitor.

### (2) Therapeutic agent for diseases

According to the present invention, a substance capable of inhibiting the binding between PTHrP and a receptor thereof can be used as a therapeutic agent for diseases attributable to vascular relaxation, in particular, to lymph duct relaxation or to medullary arteriole relaxation. Examples of the diseases attributable lymph duct relaxation include lymph duct cancer metastasis, lymphedema, lymphadenitis, and intestinal lymphangiectasis. In addition, the onset of either one or combination of these diseases, or a complication thereof with other diseases may be included. Examples of the diseases attributable to medullary arteriole relaxation include bone cancer metastasis, osteonecrosis, osteomyelitis, osteoporosis, Paget's disease of the bone, rheumatoid arthritis, osteomalasia. In addition, the onset of either one or combination of these diseases, or a complication thereof with other diseases may be included. Aside from the above-mentioned diseases, examples of the diseases attributable to vascular relaxation include hypotension, peripheral circulartory failure, and multiple organ failure, and the onset of either one or combination of these diseases, or a complication thereof with other diseases may also be included.

In the present invention, a therapeutic agent comprising as an active ingredient a substance (anti-PTHrP(1-34) antibody, L-NAME, and others) capable of inhibiting the binding between PTHrP and a receptor thereof may be administered orally or parenterally, while parenteral administration is preferable. Dosage forms thereof are the same as described above. An effective single dose may be selected within the range from 0.001 to 1000 mg per kilogram of the body weight. Alternatively, the dose per patient may be selected in the range from 0.01 to 100000 mg/body, preferably 0.1 to 10000 mg/body, more preferably 0.5 to 1000 mg/body, and much more preferably 1 to 100 mg/body. However, the therapeutic agent is not limited to these dosage ranges.

The therapeutic agent of the present invention may be formulated by any conventional method, which is also the same as described above.

### 5. Preparation of a perfused medullary arteriole specimen

The present invention also provides a method for preparing a perfused medullary arteriole specimen. The method is conducted by the following technique.

### (1) Collection of bone

Laboratory animals such as mice, rats, guinea pigs, rabbits, sheep, goats, dogs, cats, monkeys, and pigs (including miniature pigs) can be used as sources of bones. Also, surgical specimens extirpated from humans may be used. The type of bone is not particularly limited, and thus bone can be collected from any body part. However, femurs (thigh bones), humeri, and tibiae are preferable. In the following description, thigh bones are used.

First, an animal is treated with an anesthetic drug such as pentbarbital and sacrificed, and all the skin, muscle, and ligament attached to the thigh are removed so as to expose and extirpate the thigh bone. The extirpated thigh bone is preserved in a chilled Krebs solution (at approximately 4°C) until use.

Next, strength is applied along the long axis direction of the thigh bone using mosquito forceps so as to crack the thigh bone along the long axis. Then, the cracked thigh bone is placed into a petri dish (containing Krebs solution at approximately 4°C). Meanwhile, it is preferable to coat the petri dish with SILIGUARD (tradename: rubber-like silicon) in order to facilitate holding and fixing the bone.

### (2) Extirpation of medullary arteriole

With respect to the thigh bone prepared in the process (1), a thigh bone is removed under a stereoscopic microscope using microsurgical forceps (commercially available forceps that is further sharpened by a grinder) to expose the bone marrow. Krebs solution (at approximately 4°C) is filled into a syringe (having a 27G needle attached thereto) and the inside of the bone marrow is washed with the Krebs solution so as to remove myeloid cells adhering to blood vessels. A blood vessel network can be observed through the above operation, and then arterioles are extirpated from the blood vessel network under a stereoscopic microscope using micro cusps and microsurgical forceps.

### (3) Cannulation and supplying internal pressure to the arteriole

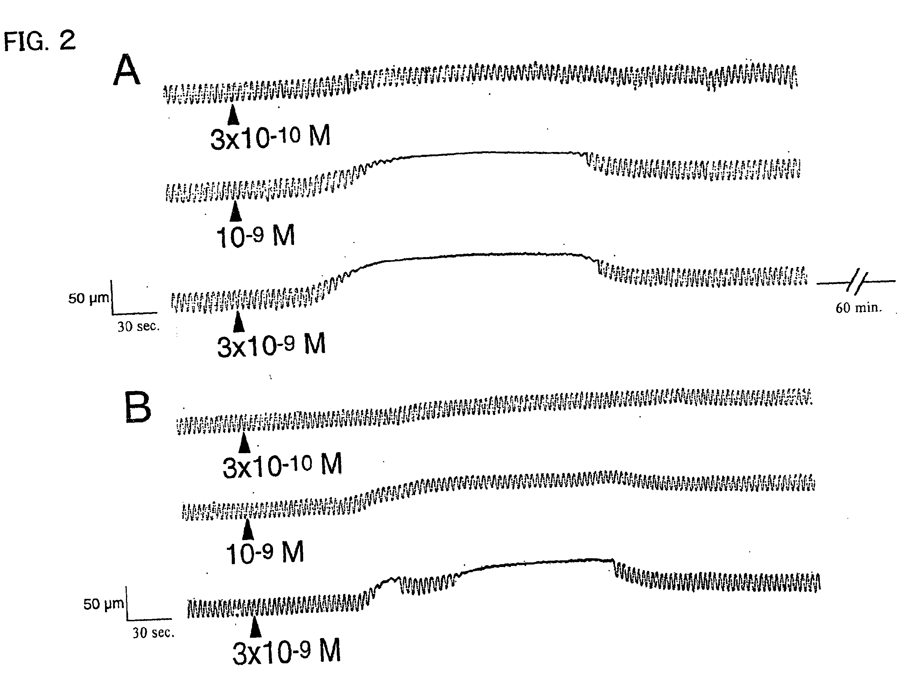

Fig. 2 shows an outline of the operation to prepare a perfused medullary arteriole specimen according to the present invention. The following operation is preferably preformed under a microscope 1 as shown in Fig. 2.

In Fig. 2, the operation to prepare the specimen starts with placing the above-extirpated arteriole 10 into a vessel chamber 9 and cannulating the specimen.

The arteriole 10 is attached to a micro glass pipette 11 (with an outer diameter of approximately 50 µm) at an arteriole inflow path side, and then tied and fixed using a nylon thread (approximately 10 µm). Next, through a Tygon tube 13 to be connected to the arteriole inflow path, a hydrostatic pressure is supplied with a syringe 15 so as to wash out and remove blood contained from the arteriole lumen. Examples of the washing solution to be used include Krebs solution and physiological saline.

With the hydrostatic pressure applied, the arteriole 10 is attached to a micro glass pipette 12 (with an outer diameter of approximately 50 µm) at an arteriole outflow path side, and then tied and fixed using a nylon thread (approximately 10 µm).

A three way stopcock 17 is attached to a Tygon tube 14 to be connected to the arteriole outflow path so as to close the outflow path. In addition, the specimen is extended in a body length direction by a manipulator 18 and fixed. Meanwhile, the Tygon tube 13 to be connected to the arteriole inflow path is connected to the syringe 15 and the internal pressure of the specimen during specimen preparation can be maintained by raising or lowering the syringe. The specimen has an internal pressure of 20 to 60 mmHg, preferably 40 mmHg.

PSS solution 22 is supplied to the vessel chamber 9 as a perfusate via a conveying tube 19 by a pump 21. Therefore, the exposed side of the perfused medullary arteriole specimen as prepared above is continuously perfused (12 ml/min.) with Krebs solution, and the perfusate is collected and discarded by a water jet aspirator. A mixed gas containing 95% oxygen and 5% carbon dioxide is supplied to the Krebs solution 22 from a mixed gas cylinder 23.

The Krebs solution 22 supplied from the pump 21 is adjusted to have a predetermined temperature by a heat exchanger 20. Krebs solution 22 is controlled to maintain a pH of 7.35 to 7.45, preferably 7.4, and a temperature of 36.5 to 37.5°C, preferably 37°C. A drug having a predetermined concentration is administered to the exposed area by the same operation as the perfusion method.

According to the present invention, the perfused medullary arteriole specimen is allowed to stand on a stage 2 of the microscope 1 and therefore microscopic observation is available. Further, a film of the specimen can be made by a video camera (also referred to as video microscope system). In Fig. 2, a CCD camera 3 is a camera for taking pictures of the specimen image magnified by microscope 1. A image of the perfused specimen of medullary arteriole is taken with the CCD camera 3 through a lens 16 of the microscope 1. A signal from the CCD camera 3 is inputted into a VCR 4 (a recording device of pictures taken with a camera), and a signal from the VCR 4 is inputted into a video caliper 5, a device for measuring inner diameter. The video caliper 5 was developed by applying edge detection system (a system for measuring diameters by determining peripheral parts of an image based on luminance changes and theshold values), and time-series changes of inner diameter can be measured automatically. In addition, it has a resolution of 0.4 µm. The information from the video caliper 5 is outputted to a chart recorder 7 and a personal computer 8 simultaneously, thereby recording changes of specimen image and actual measurement values of inner diameter changes. Also, the images displayed on a monitor 6 can be observed.

### 6. Screening method of therapeutic agents

According to the present invention, a test substance to be screened is added to the specimen as prepared above so as to detect the presence or absence of pharmacologic action concerning contraction or relaxation, and thereby screening whether or not the test substance is suitable as a relaxation or contraction agent for vessels or lymph ducts.

### BRIEF DESCRIPTION OF THE DRAWINGS

Fig. 1 is a schematic view of an apparatus for preparing a prefused medullary arteriole specimen.
Fig. 2 is a view illustrating typical responses by PTHrP(1-34) against spontaneous lymph duct contraction, and tachyphylaxis action by PTHrP(1-34).
Fig. 3 is a view illustrating a typical response by PTHrP(1-34) (3×10⁻⁹M) against spontaneous contraction of an extirpated mouse lymph duct, and an impact of NO synthesis inhibitor L-NAME (3×10⁻⁵M) and an impact of simultaneous administration of L-NAME and L-arginine (10⁻³M) against spontaneous contraction of the lymph duct by PTHrP(1-34) (3×10⁻⁹M).
Fig. 4 is a view illustrating the impact of L-NAME(3×10⁻⁵M) and the impact of simultaneous administration of L-NAME (3×10⁻⁵M) and L-arginine (10⁻³M) against spontaneous contraction of lymph duct by PTHrP(1-34).
Fig. 5 is a view illustrating a typical response by PTHrP(1-34) (3×10⁻⁹M) against spontaneous contraction of an extirpated mouse lymph duct, and the impact of an ATP-sensitive K⁺ channel inhibitor, glibenclamide (10⁻⁶M), against spontaneous contraction of the lymph duct by PTHrP(1-34) (3×10⁻⁹M).
Fig. 6 is a view illustrating the impact of glibenclamide (10⁻⁶M) against spontaneous contraction of the lymph duct by PTHrP(1-34).
Fig. 7 is a view illustrating the impact of a PTH receptor antagonist, PTHrP(7-34) (10⁻⁷M) against spontaneous contraction of the lymph duct by PTHrP(1-34).
Fig. 8 is a photograph showing a network of medullary blood vessels.
Fig. 9 is a photograph showing dilation response of an extirpated rat medullary arteriole by PTHrP(1-34).
Fig. 10 is a view illustrating dose-dependent dilation response of the extirpated rat medullary arteriole by PTHrP(1-34).
Fig. 11 is a group of photographs showing relaxation effect on an extirpated human medullary arteriole by PTHrP(1-34).

1. microscope
2. stage
3. CCD camera
4. video recorder-recording device
5. video caliper
6. monitor
7. chart recorder
8. personal computer
9. vessel chamber
10. arteriole
11. glass pipet
12. glass pipet
13. Tygon tube
14. Tygon tube
15. syringe
16. lens
17. three way stopcock
18. manipulator
19. conveying tube
20. heat exchanger
21. pump
22. Krebs solution
23. mixed gas cylinder

### BEST MODE FOR CARRYING OUT THE PRESENT INVENTION

Hereinafter, the present invention will be described in detail by Examples, however, the technical scope of the present invention is not limited by these Examples.

### [Example 1] Test for inhibition of spontaneous contraction of lymph duct

### (1) Method

Pentbarbital sodium (50mg/kg) was intraperitoneally administered to DDY mice (male, 5 to 6 weeks old) so as to be anesthetized and sacrificed, and an efferent lymph duct of an iliac lymph node (with a maximum inner diameter of 300 µm) was extirpated. The extirpated lymph duct was preserved in a chilled Krebs solution (120mM NaCl, 5.9mM KCl, 2.5mM CaCl₂, 1.2mM MgSO₄, 1.2mM NaH₂PO₄, 5.5mM glucose, 25.0mM NaHCO₃) at 4°C.

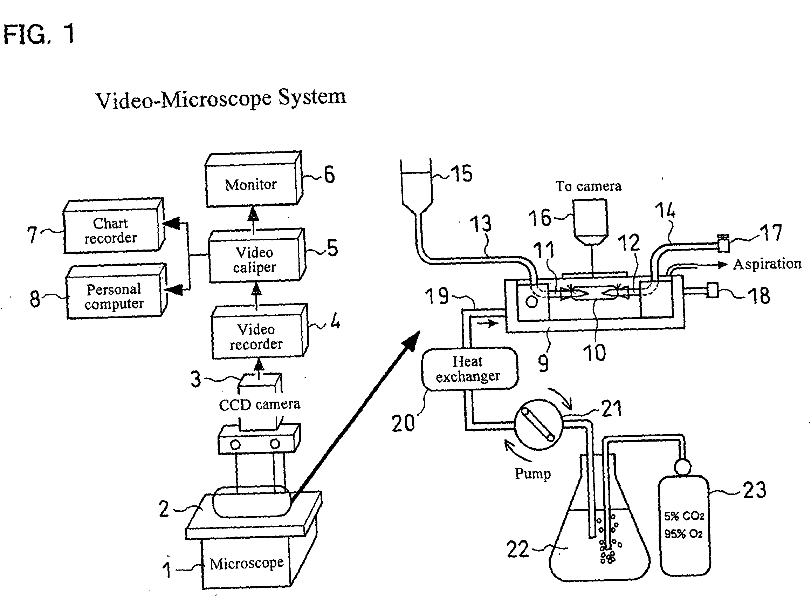

The inner diameter of the perfused extirpated mouse lymph duct specimen, as obtained above, was measured using a video microscope system (Fig. 1). An internal pressure of 3 to 4 cm H₂O was supplied to the specimen to induce spontaneous contraction.

Effects of PTHrP(1-34) on spontaneous contraction of the lymph duct were examined and compared with a control, in the presence of NO synthesis inhibitor (L-NAME, 3×10⁻⁵ M), L-arginine (10⁻³ M) as a substrate of L-NAME+NO, glibenclamide 10⁻⁶ M as an ATP-sensitive K⁺ channel inhibitor, and RTHrP(7-34) as a PTH receptor antagonist.

### (2) Results

Fig. 2 shows examples of typical responses obtained while examining the different effects of PTHrP(1-34) (3×10⁻¹⁰M to 3×10⁻⁹M) on spontaneous contraction of the lymph duct. The fluctuation of the baseline as shown in Fig. 2 corresponds to inner diameter changes of the spontaneous contraction of the lymph duct, and upper crests and lower troughs represent dilation and contraction, respectively.

The extirpated mouse lymph duct generated stable spontaneous contractions (approximately 15 times per minute) wherein the lymph duct had a largest diameter of 150 to 300 µm and the contraction had an amplitude of 20 to 30 µm according to the supplied inner pressure. In Fig. 2A which illustrates a case of 3×10⁻¹⁰ of PTHrP(1-34), there was 15 times/minute of spontaneous contraction before drug administration, and frequency of spontaneous contraction was decreased to 13 times/minute after drug administration. Therefore, PTHrP(1-34) decreased contraction frequency, that is induced a negative chronotropic action. Further, when the concentration of PTHrP(1-34) was increased (10⁻⁹ M, 3×10⁻⁹ M), spontaneous contractions, in addition to dilation responses, were stopped remarkably. The same specimen was washed for 1 hour, and thereafter dose response curve was examined in the same manner as those in Fig. 2A. The results are shown in Fig. 2B. As shown in Fig. 2B, it turned out that PTHrP(1-34) induced remarkable tachyphylaxis.

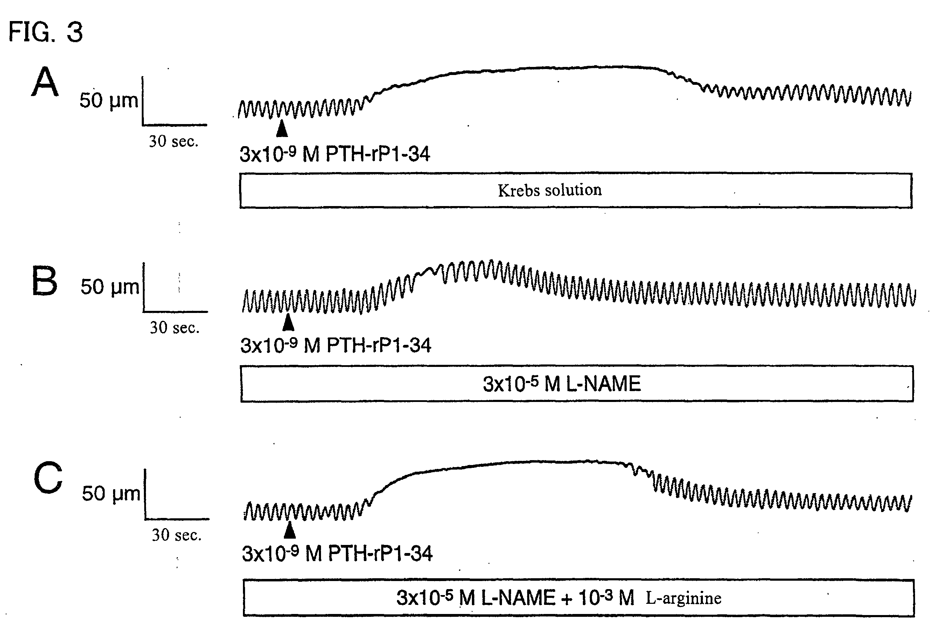

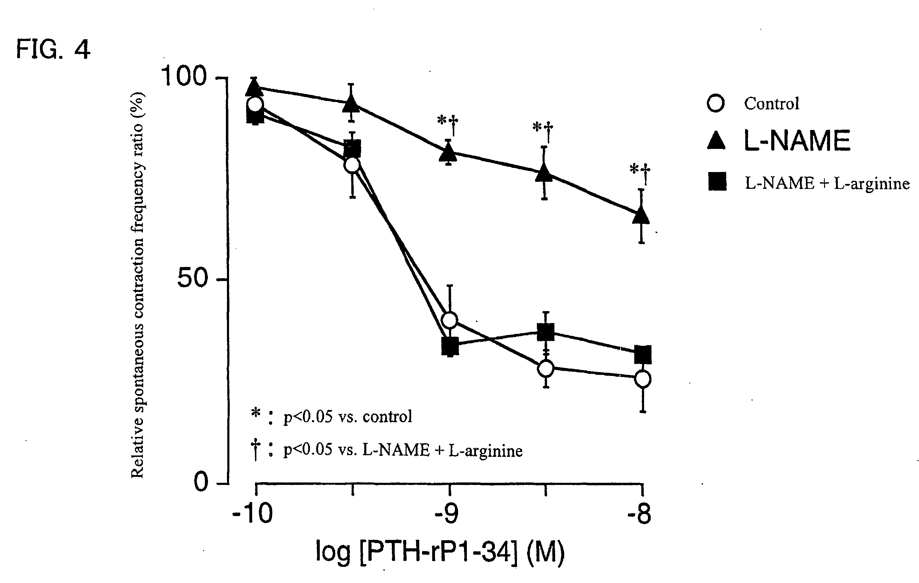

Fig. 3 shows an example of a typical response obtained when NO-involvement was examined against PTHrP(1-34) (3×10⁻⁹ M) inhibition of spontaneous contraction of the lymph duct. Inhibition of spontaneous lymph duct contraction by PTHrP(1-34), which is shown in Fig. 3A, was cancelled out in the presence of 3×10⁻⁵ M of L-NAME, an NO synthesis inhibitor (Fig. 3B). Further, simultaneous treatment of L-NAME and 10⁻³ M of L-arginine suppressed the cancellation effect of L-NAME, and thus the same reaction as the control (without addition of L-NAME and L-arginine) (Fig. 3C) was obtained. Four measurements were conducted for each treated group with these drugs. The results thereof are shown in Fig. 4. In Fig. 4, the expression "relative spontaneous contraction frequency ratio" means a percentage of spontaneous contraction frequency after administration of drug relative to spontaneous contraction frequency. Thus, as the percentage is smaller, the spontaneous contraction frequency is lower (that is, being relaxed). In the case of the control to which L-NAME and L-arginine were not added, increasing PTHrP(1-34) relaxed the lymph duct (Fig. 4, ο). However, this relaxation was significantly cancelled out in the presence of L-NAME (Fig. 4, ▲), thereby causing spontaneous contraction. Further, simultaneous treatment of L-NAME and L-arginine significantly suppressed the cancellation effect of L-NAME (Fig. 4, ■), and then the same level of relaxation as the control was observed.

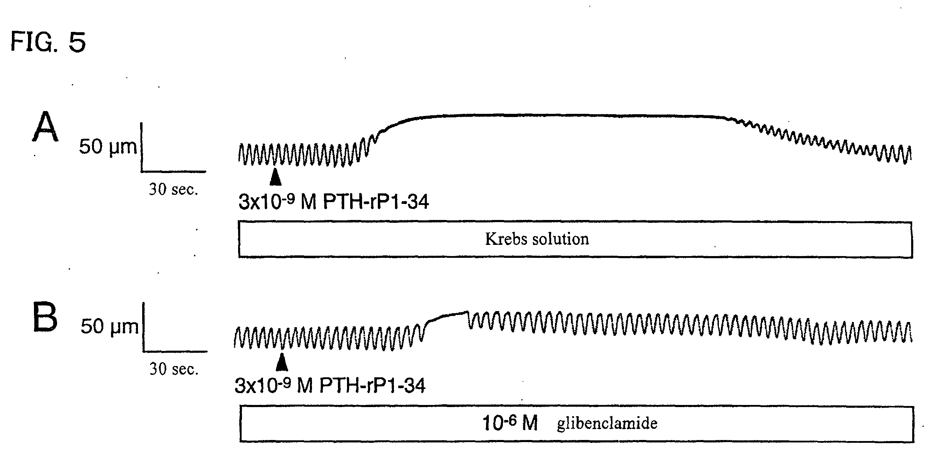

Fig. 5 shows an example of a typical response obtained during the examination on the involvement of an ATP-sensitive K⁺ channel against spontaneous contraction inhibition of the lymph duct by 3×10⁻⁹M of PTHrP(1-34). The lymph duct relaxation, shown in Fig. 5A, as caused by PTHrP(1-34), was cancelled out due to the presence of selective ATP-sensitive K⁺ channel inhibitors, glibenclamide (10⁻⁶ M) (Fig. 5B).

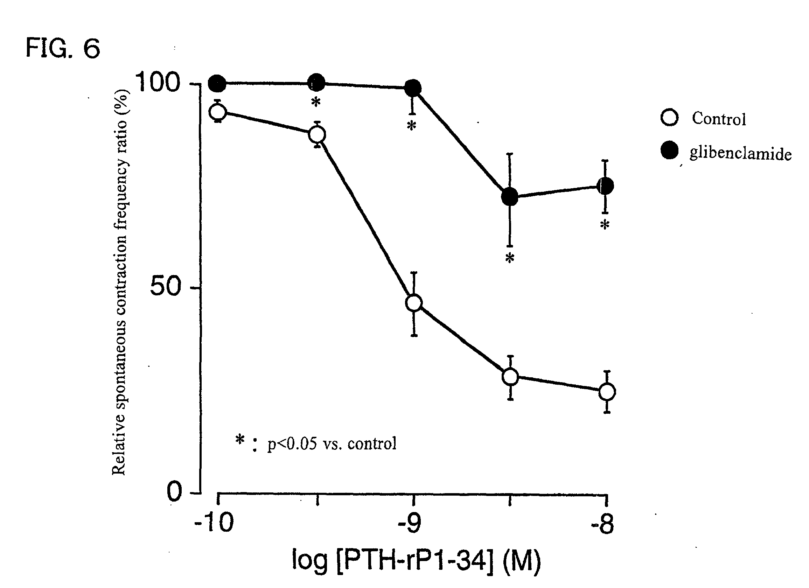

Four measurements were conducted for each treated group with these drugs. The results thereof are shown in Fig. 6. The lymph duct relaxation caused by PTHrP(1-34), which was observed in the control (without the presence of glibenclamide) was significantly cancelled out because of the presence of glibenclamide (Fig. 6, ●).

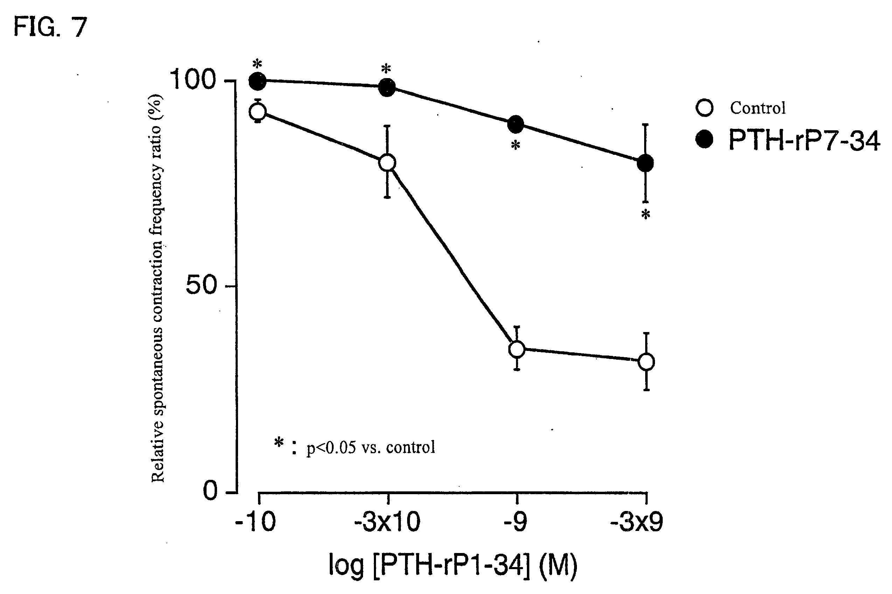

Fig. 7 shows the results of four individual examples tested for effects of a PTH/PTHrP receptor antagonist, i.e. PTHrP(7-34) (10⁻⁷M) on the spontaneous contraction of the lymph duct by PTHrP(1-34).

The lymph duct relaxation by 10⁻¹⁰ to 3×10⁻⁹ M of PTHrP(1-34) was significantly cancelled out by 10⁻⁷ M of PTHrP(7-34) (Fig. 7, ●).

According to the above results, the following points were concluded.
1) PTHrP(1-34) inhibits spontaneous contraction of the extirpated mouse lymph duct.
2) Spontaneous lymph duct contraction inhibition by PTHrP(1-34) is expressed via a PTH/PTHrP receptor.
3) Intrinsic NO and ATP sensitive K⁺ channel are involved in the inhibition of spontaneous lymph duct contraction by PTHrP(1-34).

### [Example 2] Preparation of a prefused specimen

### (1) Material and method

### ① Method for extirpating specimen

Wistar rats (male, 5 to 6 weeks old) were anesthetized with pentbarbital (50 mg/ml of intraperitoneal administration) and sacrificed. All the skin, muscle, and ligament attached to the thigh bones were removed so as to expose and extirpate the thigh bones. The extirpated thigh bones were preserved in Krebs solution (at approximately 4°C) for approximately 30 minutes. Strength was applied along the long axis direction of the thigh bone using mosquito forceps so as to crack the thigh bone along the long axis. Then, the cracked thigh bone was place into a petri dish (containing Krebs solution at approximately 4°C) which was coated with SILIGUARD. Under a stereoscopic microscope, the thigh bone was removed using microsurgical forceps (commercially available forceps that was further sharpened by a grinder) to expose the bone marrow.

Krebs solution (at approximately 4°C) was filled into a 1 ml syringe (having a 27G needle attached thereto) and the inside of the bone marrow was washed with the Krebs solution so as to remove myeloid cells adhering to blood vessels.

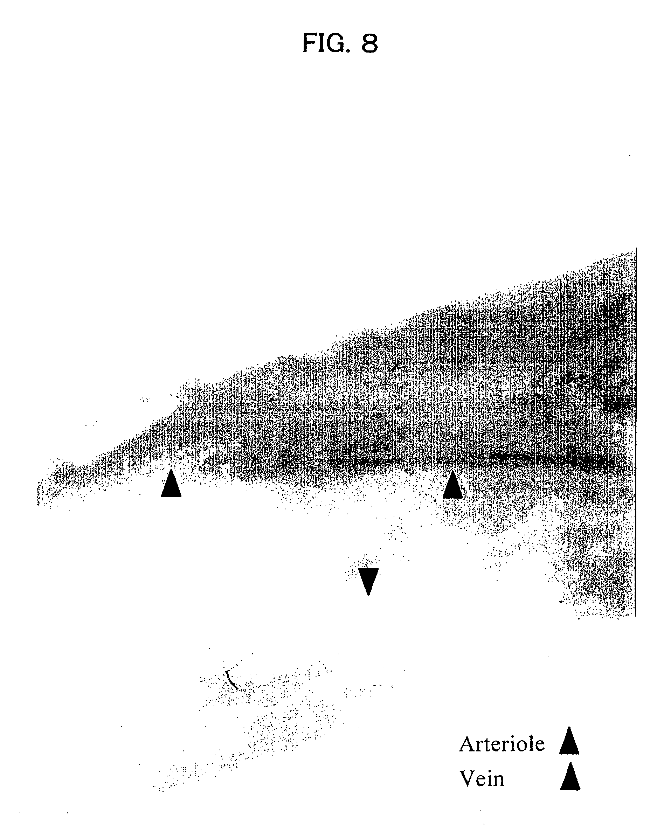

As a result, a blood vessel network was observed (Fig. 8), and arterioles indicated by black wedge-shaped arrowheads were extirpated under a stereoscopic microscope using micro cusps and microsurgical forceps.

### ② Method for preparing a perfused specimen

The extirpated medullary arteriole was fixed in an apparatus for preparing a perfused specimen (Fig. 1, vessel chamber 9), and a hydrostatic pressure was supplied thereto so as to wash and remove blood from the arteriole lumen. The inner pressure of the specimen was kept at 40 mmHg during the experiment and the exposed side of the specimen was continuously perfused (12 ml/min) with Krebs solution which was kept at 37°C and pH of 7.4.

### ③ Method for measuring inner diameter changes of arteriole

An image of the diameter of the specimen was captured through a object lens, a taking lens, and a CCD camera.

### (2) Results

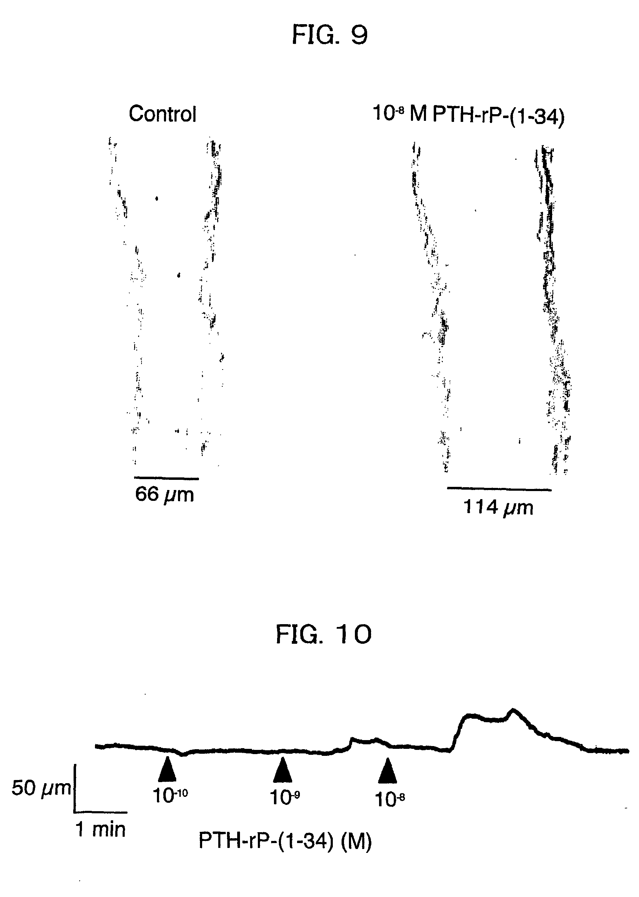

An image of an medullary arteriole of rat thigh bone before the administration of PTHrP(1-34)(10⁻⁸M) is shown in the left side of Fig. 9, . This specimen exhibited inner pressure-dependent and temperature-dependent myogenic spontaneous contraction like medullary arterioles of other organs (heart, brain, skeletal muscle, and intestinal membrane). This specimen exhibited a myogenic contraction of 66 µm from a passive blood vessel diameter of 120 µm after approximately 1 hour of heating. An image of the medullary arteriole of rat thigh bone exhibiting a maximum dilation (relaxation) obtained after the administration of PTHrP(1-34) (10⁻⁸M) is shown in the right of Fig. 9 is a. The administration of PTHrP(1-34) (10⁻⁸M) caused the inner diameter to be dilated (relaxed) from 66 µm to 114 µm.

Fig. 10 shows a PTHrP(1-34) (10⁻¹⁰ to 10⁻⁸M) dose dependent curve of the medullary arteriole of rat thigh bone. In the figure, the vertical axis and horizontal axis indicate changes of blood vessel diameter (upper direction from the baseline indicates dilation) and elapsed time, respectively. pTHrP(1-34) induced dilation response at a dose of 10⁻⁹M, and its effect was dose dependent. Further, after a maximum dilation response was obtained at the dose of 10⁻⁸M, the specimen was recovered to the same state as before drug administration.

### [Example 3] Examination of relaxation effects on an extirpated human medullary arteriole

### (1) Material and method

### ① Method for extirpating a specimen

Resistance vessel extirpated from femoral cancellous bone and the inside of femoral bone marrow during an artificial joint replacement surgery of a human (malum coxae, female, 55 years old) was used as a sample of human medullary arteriole. The extirpated femoral concellous bone and femoral bone marrow were preserved in a chilled Krebs solution (at 4°C or lower), and thereafter used in the following *in vitro* experiment.

### ② Method for preparing a perfused specimen

A perfused specimen from this sample was prepared in the same manner as the perfused specimen of the extirpated mouse miniature lymph duct in Example 1. The Krebs solution used in the experiment had an oxygen partial pressure of 21%, PO₂ of 150mmHg or less, while the internal pressure supplied to the human medullary arteriole was kept at 55 mmHg.

### ③ Method for measuring inner diameter changes of arteriole

An image of the diameter of the specimen was captured through an object lens, a taking lens, and a CCD camera in the same manner as Example 2.

### (2) Results

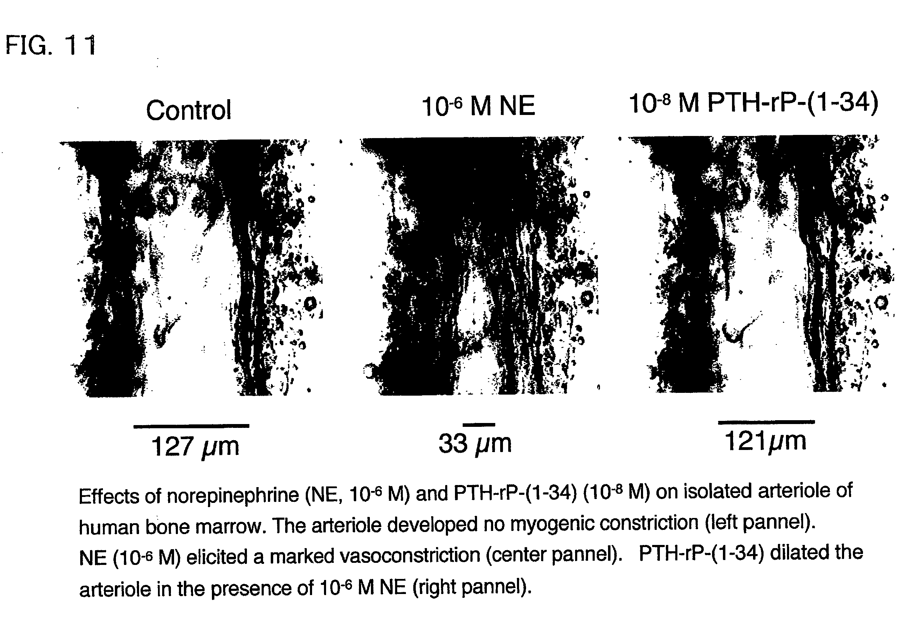

With respect to the perfused specimen as prepared above, relaxation effect of PTHrP(1-34)(10⁻⁸M) in the presence of vasoconstricting norepinephrine (NE, 10⁻⁶M) was examined. The results are shown in Fig. 11. A photograph of Fig. 11(a) shows a perfused specimen of a control without PTHrP(1-34) and NE. A photograph of Fig. 11(b) shows a perfused specimen in the presence of NE (10⁻⁶M). A photograph of Fig. 11(c) shows a perfused specimen in the presence of NE (10⁻⁶M) and PTHrP(1-34) (10⁻⁸M).

The perfused control specimen had an inner diameter of 127 µm. In contrast, the perfused specimen's inner diameter with the presence of NE (10⁻⁶M) contracted to 33 µm. This indicates that NE (10⁻⁶M) has a remarkable vasocontrictive effect on human medullary arteriole. On the other hand, addition of PTHrP(1-34) (10⁻⁸M) in the presence of NE (10⁻⁶M) caused the inner diameter of the perfused specimen to relaxe to 121 µm, which was almost same as that of the control. According to this result, PTHrP(1-34) has a relaxation effect, in particular, on bone arterioles.

All publications, patents and patent applications cited herein are incorporated herein by reference in their entirety.

### Industrial Applicability

According to the present invention, there are provided relaxation agents, which comprise parathyroid hormone related peptides or a fragment thereof, for the vascular system, lymph ducts, and medullary arterioles, and vascular relaxation inhibitors, which comprise a substance capable of inhibiting the binding between PTHrP and a receptor thereof, for medullary arterioles and medullary arterioles.

## Claims

1. A vascular relaxation agent comprising PTHrP or a fragment thereof.

2. The vascular relaxation agent according to claim 1, wherein the agent relaxes a lymph duct.

3. The vascular relaxation agent according to claim 1, wherein the agent relaxes a medullary arteriole.

4. A therapeutic agent for a disease attributable to vascular contraction, comprising PTHrP or a fragment thereof.

5. The therapeutic agent according to claim 4, wherein the disease attributable to vascular contraction is at least one selected from the group consisting of myocardial infraction, hypertension, thrombosis, congestive heart failure, and angina pectoris.

6. A vascular relaxation inhibitor comprising a substance capable of inhibiting the binding between PTHrP and a receptor thereof.

7. The vascular relaxation inhibitor according to claim 6, wherein the inhibitor inhibits lymph duct relaxation.

8. The vascular relaxation inhibitor according to claim 6, wherein the inhibitor inhibits medullary arteriole relaxation.

9. A therapeutic agent for a disease attributable to vascular relaxation, comprising a substance capable of inhibiting the binding between PTHrP and a receptor thereof.

10. The therapeutic agent according to claim 9, wherein the disease is at least one selected from the group consisting of hypotension, peripheral circulatory failure, and multiple organ failure.

11. A therapeutic agent for a disease attributable to lymph duct relaxation, comprising a substance capable of inhibiting the binding between PTHrP and a receptor thereof.

12. The therapeutic agent according to claim 11, wherein the disease is at least one selected from the group consisting of lymph duct cancer metastasis, lymphedema, lymphadenitis, and intestinal lymphangiectasis.

13. A therapeutic agent for a disease attributable to medullary arteriole relaxation, comprising a substance capable of inhibiting the binding between PTHrP and a receptor thereof.

14. The therapeutic agent according to claim 13, wherein the disease is at least one selected from the group consisting of bone cancer metastasis, osteonecrosis, osteomyelitis, osteoporosis, Paget's disease of the bone, rheumatoid arthritis, and osteomalasia.

15. A method for preparing a perfused medullary arteriole specimen comprising the steps of:
(a) collecting bone from a laboratory animal;
(b) exposing bone marrow by stripping off a surface of the bone;
(c) extirpating a medullary arteriole from inside of the bone marrow;
(d) attaching cannulas to the extirpated arteriole; and
(e) supplying an internal pressure to the arteriole.

16. The method according to claim 15, wherein the bone is thigh bone.

17. A method for screening a medullary arteriole relaxation agent and/or relaxation inhibitor, comprising the steps of:
adding a test substance to the perfused medullary arteriole specimen obtained by the method as set forth in claim 15 or 16; and
screening the test substance using contraction or relaxation of the specimen as an index.

18. An apparatus for measuring contraction and relaxation of a vessel to be examined, comprising:
a pair of cannulas which are attached on both ends of the vessel to be examined so as to support the vessel;
internal pressure application means which supplies an internal pressure to the vessel to be examined via at least one of the cannulas;
a vessel chamber inside which the vessel supported by the pair of cannulas is placed, being filled with a perfusion liquid; and
imaging means which takes an image of the vessel inside the vessel chamber,
wherein the imaging means detects changes in the shape of the vessel supported by the pair of cannulas thereby measuring the contraction and relaxation of the vessel to be examined.

19. A method for measuring contraction and/or relaxation of a vessel to be examined, comprising the steps of:
attaching a pair of cannulas on both ends of the vessel to be examined, and supporting the vessel by the pair of the cannulas;
supplying an internal pressure to the vessel to be examined via at least one of cannulas; and
measuring contraction and/or relaxation of the vessel to be examined in a condition where the vessel supported by the pair of cannulas is immersed in a perfusion liquid filled into a vessel chamber.


SEQUENCE LISTING

<110> CHUGAI SEIYAKU KABUSIKI KAISYA

<120> VESSEL CHALASIA AGENT

<130> PH-1247-PCT

<150> JP 2000-243873

<151> 2000-08-11

<160> 3

<170> PatentIn Ver. 2.0

<210> 1

<211> 139

<212> PRT

<213> Homo sapiens

<400> 1

Ala Val Ser Glu His Gln Leu Leu His Asp Lys Gly Lys Ser Ile Gln

1               5                   10                  15

Asp Leu Arg Arg Arg Phe Phe Leu His His Leu Ile Ala Glu Ile His

            20                  25                  30

Thr Ala Glu Ile Arg Ala Thr Ser Glu Val Ser Pro Asn Ser Lys Pro

        35                  40                  45

Ser Pro Asn Thr Lys Asn His Pro Val Arg Phe Gly Ser Asp Asp Glu

    50                  55                  60

Gly Arg Tyr Leu Thr Gln Glu Thr Asn Lys Val Glu Thr Tyr Lys Glu

65                  70                  75                  80

Gln Pro Leu Lys Thr Pro Gly Lys Lys Lys Lys Gly Lys Pro Gly Lys

                85                  90                  95

Arg Lys Glu Gln Glu Lys Lys Lys Arg Arg Thr Arg Ser Ala Trp Leu

            100                 105                 110

Asp Ser Gly Val Thr Gly Ser Gly Leu Glu Gly Asp His Leu Ser Asp

        115                 120                 125

Thr Ser Thr Thr Ser Leu Glu Leu Asp Ser Arg

    130                 135

<210> 2

<211> 141

<212> PRT

<213> Homo sapiens

<400> 2

Ala Val Ser Glu His Gln Leu Leu His Asp Lys Gly Lys Ser Ile Gln

1               5                   10                  15

Asp Leu Arg Arg Arg Phe Phe Leu His His Leu Ile Ala Glu Ile His
            20                  25                  30
Thr Ala Glu Ile Arg Ala Thr Ser Glu Val Ser Pro Asn Ser Lys Pro
        35                  40                  45
Ser Pro Asn Thr Lys Asn His Pro Val Arg Phe Gly Ser Asp Asp Glu
    50                  55                  60
Gly Arg Tyr Leu Thr Gln Glu Thr Asn Lys Val Glu Thr Tyr Lys Glu
65                  70                  75                  80
Gln Pro Leu Lys Thr Pro Gly Lys Lys Lys Lys Gly Lys Pro Gly Lys
                85                  90                  95
Arg Lys Glu Gln Glu Lys Lys Lys Arg Arg Thr Arg Ser Ala Trp Leu
            100                 105                 110
Asp Ser Gly Val Thr Gly Ser Gly Leu Glu Gly Asp His Leu Ser Asp
        115                 120                 125
Thr Ser Thr Thr Ser Leu Glu Leu Asp Ser Arg Arg His
    130                 135                 140
<210> 3
<211> 173
<212> PRT

<213> Homo sapiens

<400> 3

Ala Val Ser Glu His Gln Leu Leu His Asp Lys Gly Lys Ser Ile Gln
1               5                   10                  15

Asp Leu Arg Arg Arg Phe Phe Leu His His Leu Ile Ala Glu Ile His
            20                  25                  30

Thr Ala Glu Ile Arg Ala Thr Ser Glu Val Ser Pro Asn Ser Lys Pro
        35                  40                  45

Ser Pro Asn Thr Lys Asn His Pro Val Arg Phe Gly Ser Asp Asp Glu
    50                  55                  60

Gly Arg Tyr Leu Thr Gln Glu Thr Asn Lys Val Glu Thr Tyr Lys Glu
65                  70                  75                  80

Gln Pro Leu Lys Thr Pro Gly Lys Lys Lys Lys Gly Lys Pro Gly Lys
                85                  90                  95

Arg Lys Glu Gln Glu Lys Lys Lys Arg Arg Thr Arg Ser Ala Trp Leu
            100                 105                 110

Asp Ser Gly Val Thr Gly Ser Gly Leu Glu Gly Asp His Leu Ser Asp
        115                 120                 125

Thr Ser Thr Thr Ser Leu Glu Leu Asp Ser Arg Thr Ala Leu Leu Trp
    130                 135                 140

Gly Leu Lys Lys Lys Lys Glu Asn Asn Arg Arg Thr His His Met Gln
145                 150                 155                 160
Leu Met Ile Ser Leu Phe Lys Ser Pro Leu Leu Leu Leu
                165                 170